# EUROPEAN PATENT APPLICATION

(11) **EP 4 390 954 A1**
(43) Date of publication of application: **26.06.2024**
(21) Application number: 22216058.2
(22) Date of filing: 22.12.2022
(51) Int. Cl.: G16H 20/30, G16H 40/67

(54) **INTELLIGENT DROP-OUT PREDICTION IN REMOTE PATIENT MONITORING**

(71) Applicant: Koninklijke Philips N.V., 5656 AG Eindhoven (NL)
(72) Inventor: VAN DE CRAEN, Dieter, Eindhoven (NL); PIJL, Marten, Eindhoven (NL)
(74) Representative: Philips Intellectual Property & Standards

(57) **Abstract**

The present disclosure is directed to methods and systems for predicting patient dropout from a remote patient monitoring (RPM) program, as well as root dropout causes, based on clinical features using a dropout prediction engine. As described herein, the methods and systems address the clinical challenge of early detection of dropout risk of patients from these virtual care programs through a data-driven approach that accurately identifies the likely root cause(s) of the dropout and enables the prevention of the dropout by applying timely interventions targeting the root causes of the dropout. As a result, dropout prevention effectuated through targeted interventions will promote continued engagement with virtual care, thereby leading to lower costs of care, better health outcomes, and better patient and staff experience.

## Description

### FIELD OF THE DISCLOSURE

The present disclosure relates generally to methods and systems for predicting dropout from a remote patient monitoring program, and more specifically to methods and systems for predicting dropout and root causes for the dropout.

### BACKGROUND

Widespread adoption of electronic health records has enabled automated data capturing and propelled predictive risk modeling in a variety of respects and across many different cohorts. In certain settings, risk modeling has become an essential pillar for measuring outcomes and other benchmarking. These and other technological advancements (e.g., advances in monitoring hubs, smartphone platforms, sensor technologies, cellular networks, cloud computing, and mobile and wearable medical devices) have removed many of the technical barriers to successful remote patient monitoring (RPM) and remote patient healthcare in general. Further, the rising geriatric population and the growing need to expand healthcare access, cost benefits of telehealth and RPM, benefits of RPM to reduce the burden on medical resources, advancements in telecommunications, growing incidences of chronic diseases, and increasing investments in telehealth and RPM are the major factors driving the growth of this market. As a result of these technological innovations and market factors, adoption and scaling of RPM has greatly accelerated.

In particular, RPM programs provide care teams with the tools they need to remotely track the health of their patients at home, collaborate with the patients' doctors and help detect problems before they lead to readmissions. Providers can assign patients to specific care protocols and interventions that are tailored to condition or acuity level, which can include measuring vital signs, completing surveys, watching educational videos and participating in video visits with the virtual care team. However, challenges towards successful deployment of RPM solutions remain and a key remaining challenge is that a substantial portion of patients will drop out early of the program.

### SUMMARY OF THE DISCLOSURE

According to an embodiment of the present disclosure, a method for predicting dropout risk for a patient under remote monitoring using a dropout prediction system is provided. The method comprises: obtaining, from an electronic patient records database, a plurality of medical records for a patient under remote monitoring by a care provider; extracting, from the plurality of medical records for the patient under remote monitoring by the care provider, a plurality of dropout prediction features for the patient; generating, using a dropout prediction engine, a dropout risk score for the patient based on the plurality of dropout prediction features; determining, using an engagement recommendation engine, a potential dropout cause based on at least the plurality of dropout prediction features; determining, using the engagement recommendation engine, a recommended engagement action, wherein the recommended engagement action is intended to prevent dropout of the patient from remote monitoring by the care provider; and presenting, via a care provider interface, the recommended engagement action to a care team member of the care provider.

In an aspect, the dropout prediction engine comprises a trained dropout prediction model, the trained dropout prediction model being trained by a machine learning algorithm on a training dataset that comprises a plurality of medical records for a plurality of historical patients.

In an aspect, the potential dropout cause is determined by evaluating a feature value contribution for one or more dropout prediction features, the feature value contributions being determined using a Shapley values algorithm.

In an aspect, the plurality of medical records for the patient under remote monitoring include at least one of: identification information for the patient; socioeconomic information for the patient; medical history for the patient; treatment history for the patient; medical directives for the patient; and one or more physiological measurements taken from the patient.

In an aspect, the plurality of medical records for the patient under remote monitoring further includes at least one of: virtual care solution usage information; a technology affinity measured for the patient; and feedback information from one or more historical or concurrent patients.

In an aspect, the dropout risk score generated for the patient is a likelihood that the patient will fail to meet a participation threshold set by a third party.

In an aspect, the third party is an insurance company and the participation threshold determines whether the remote monitoring of the patient is covered under an insurance plan associated with the patient.

In an aspect, the method further comprises: determining a phenotype for the patient under remote monitoring based on one or more of the dropout prediction features, the dropout risk score generated for the patient, and the potential dropout cause determined for the patient, wherein the phenotype for the patient indicates a subgroup of similar historical patients; wherein the recommended engagement action is determined at least in part based on the phenotype for the patient.

According to another embodiment of the present disclosure, a dropout prediction system configured to predict a dropout risk for one or more patients undergoing remote patient monitoring by a care provider is provided. The system comprises: an electronic patient records database comprising a plurality of medical records for the one or more patients under remote monitoring by the care provider; a dropout prediction database comprising a repository of root dropout causes and a library of engagement actions; a dropout prediction engine configured to generate one or more dropout risk scores for the one or more patients; an engagement recommendation engine configured to determine one or more potential dropout cause and one or more recommended engagement actions for the one or more patients; a care provider interface configured to present one or more dropout risk scores generated for the one or more patients, the one or more potential dropout causes for the one or more patients, and/or the one or more recommended engagement actions for the one or more patients; and one or more processors configured to: (i) obtain, from the electronic patient records database, a plurality of medical records for at least a first patient; (ii) extract, from the plurality of medical records, a plurality of dropout prediction features for at least the first patient; (iii) generate, using the dropout prediction engine, a dropout risk score for at least the first patient based on the plurality of dropout prediction features; (iv) determine, using the engagement recommendation engine, a potential dropout cause for at least the first patient based on at least the plurality of dropout prediction features; (v) determine, using the engagement recommendation engine, a recommended engagement action, wherein the recommended engagement action is intended to prevent dropout of at least the first patient from remote monitoring by the care provider; and (vi) present, via the care provider interface, present the dropout risk score generated for at least the first patient, the potential dropout cause for at least the first patient, and/or the recommended engagement action for at least the first patient.

In an aspect, the dropout prediction engine comprises a trained dropout prediction model, the trained dropout prediction model being trained by a machine learning algorithm on a training dataset that comprises a plurality of medical records for a plurality of historical patients.

In an aspect, each potential dropout cause is determined by evaluating a feature value contribution for one or more dropout prediction features, the feature value contributions being determined using a Shapley values algorithm.

In an aspect, the plurality of records for the patient under remote monitoring by the care provider include at least one of: identification information for the patient; socioeconomic information for the patient; medical history for the patient; treatment history for the patient; medical directives for the patient; and one or more physiological measurements taken from the patient.

In an aspect, the plurality of records for the patient under remote monitoring by the care provider further includes at least one of: virtual care solution usage information; a technology affinity measured for the patient; and feedback information from one or more historical or concurrent patients.

In an aspect, the engagement recommendation engine is further configured to determine a phenotype for the one or more patients, each phenotype indicating a subgroup of similar historical patients; and wherein the one or more processors are further configured to: (vii) determine, using the engagement recommendation engine, a phenotype for at least the first patient based on one or more of the dropout prediction features of at least the first patient, the dropout risk score generated for at least the first patient, and the potential dropout cause determined for at least the first patient; and (viii) determine a recommended engagement action based at least in part on the phenotype determined for at least the first patient.

In an aspect, the dropout risk score generated for at least the first patient is a likelihood that the patient will fail to meet a participation threshold set by a third party, the third party being an insurance company and the participation threshold determining whether the remote monitoring of at least the first patient is covered under an insurance plan associated with at least the first patient.

These and other aspects of the various embodiments will be apparent from and elucidated with reference to the embodiments described hereinafter.

### BRIEF DESCRIPTION OF THE DRAWINGS

In the drawings, like reference characters generally refer to the same parts throughout the different views. Also, the drawings are not necessarily to scale, emphasis instead generally being placed upon illustrating the principles of the various embodiments.
FIG. 1 is a flowchart illustrating a method for predicting dropout risk for a patient under remote monitoring according to aspects of the present disclosure.
FIG. 2A is a flowchart illustrating further features of the method for predicting dropout risk for a patient under remote monitoring according to aspects of the present disclosure.
FIG. 2B is a flowchart illustrating yet further features of the method for predicting dropout risk for a patient under remote monitoring according to aspects of the present disclosure.
FIG. 3 is a diagram illustrating the deployment of a dropout prediction system according to aspects of the present disclosure.
FIG. 4 is a block diagram illustrating the dropout prediction system according to aspects of the present disclosure.

### DETAILED DESCRIPTION OF EMBODIMENTS

The present disclosure is directed to methods and systems for predicting patient dropout from a remote patient monitoring (RPM) program, as well as root dropout causes, based on clinical features using a dropout prediction engine. As described herein, the methods and systems address the clinical challenge of early detection of dropout risk of patients from these virtual care programs through a data-driven approach that accurately identifies the likely root cause(s) of the dropout and enables the prevention of the dropout by applying timely interventions targeting the root causes of the dropout. As a result, dropout prevention effectuated through targeted interventions will promote continued engagement with virtual care, thereby leading to lower costs of care, better health outcomes, and better patient and staff experience.

The embodiments and implementations disclosed or otherwise envisioned herein can be utilized with any virtual patient care system or platform, including but not limited to clinical decision support tools, among other systems. For example, one application of the embodiments and implementations herein is to improve systems such as, e.g., the Philips^{®} BioTel, Philips^{®} Engage, SRC digital and remote care solutions, and the like, among many others. However, the disclosure is not limited to these devices or systems, and thus disclosure and embodiments disclosed herein can encompass any device or system capable of remote patient monitoring.

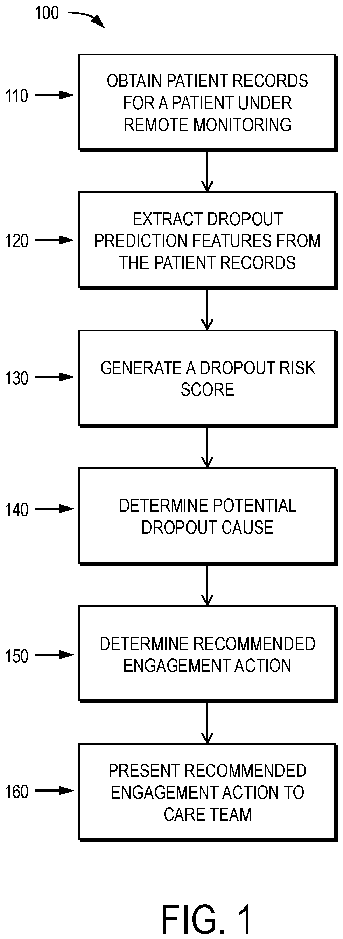

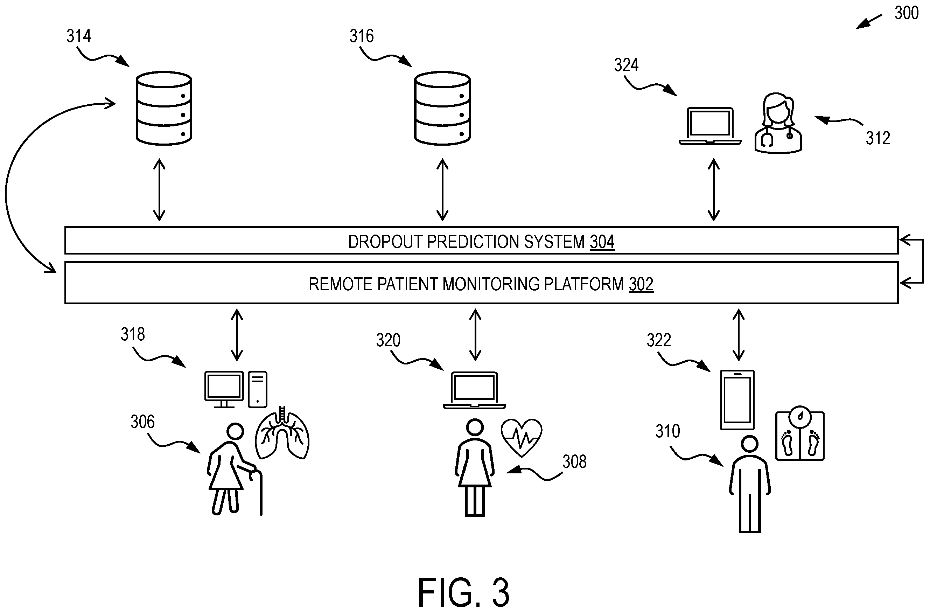

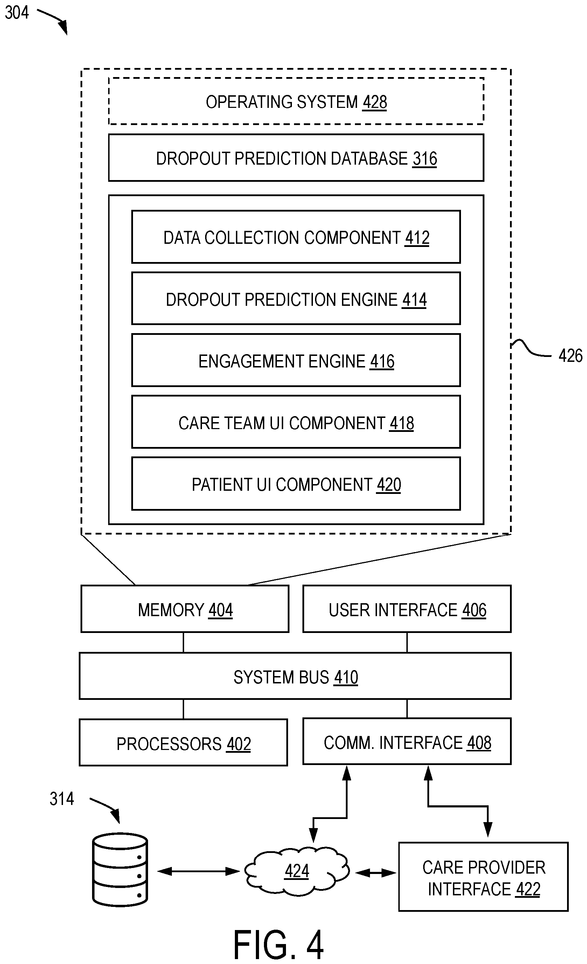

Turning to FIG. 1, a flowchart of a method 100 for predicting dropout risk for a patient under remote monitoring using a dropout prediction system is illustrated according to aspects of the present disclosure. The dropout prediction system can be any of the systems described or otherwise envisioned herein. For example, as discussed in greater detail below, the dropout prediction system (e.g., dropout prediction system 304 shown in FIGS. 3 and 4) can be configured to predict a dropout risk for one or more patients undergoing remote patient monitoring (RPM) by a care provider. In embodiments, the dropout prediction system can include: an electronic patient records database comprising a plurality of medical records for the one or more patients under remote monitoring by the care provider; a dropout prediction database comprising a repository of root dropout causes and a library of engagement actions; a dropout prediction engine configured to generate one or more dropout risk scores for the one or more patients; an engagement recommendation engine configured to determine one or more potential dropout causes and one or more recommended engagement actions for the one or more patients; a care provider interface configured to present one or more dropout risk scores generated for the one or more patients, the one or more potential dropout causes for the one or more patients, and/or the one or more recommended engagement actions for the one or more patients; and one or more processors configured to performed one or more steps of the methods (e.g., method 100) described herein.

At a step 110, the method 100 includes obtaining a plurality of records for a patient in an RPM program. In some embodiments, the plurality of records for the patient may be obtained by the dropout prediction system 304. In further embodiments, the plurality of records for the patient may be obtained from an electronic patient records database where all the available patient data relevant for dropout prediction and root cause prediction are stored and/or accessible. For example, the electronic patient records database may include physiologic, diagnosis, and treatment information (collectively, "medical information" or "medical data") for a plurality of patients under one or more RPM programs administered by one or more care providers. That is, the electronic patient records database can comprise a plurality of healthcare-related records for a plurality of patients, including historical patients and/or patients of one or more current RPM programs.

In some embodiments, the electronic patient records database can include self-reported data on medical conditions (existing or new), physical health, and mental health for the patient. Put another way, the patient records electronic database can include medical data generated and reported by the patient, including but not limited to, updated physiological measurements and test results.

In further embodiments, the electronic patient records database can also include patterns of use of the virtual care system used in the patient's RPM program, including but not limited to, virtual care platforms, virtual health assistants, electronic and/or outpatient medical devices, virtual visit systems, medical appointment scheduling systems, and the like. In embodiments, the electronic patient records database includes patterns of medical data (e.g., test results, hospital visits, provider interactions), as well as other engagement-related features such as medication adherence.

In embodiments, the electronic patient records database may also include subjective data that is collected, self-reported, and/or assessed over time, including but not limited to, the patient's comfortability and/or affinity with technology.

In specific embodiments, the plurality of medical records stored in the electronic patient records database 402 can include one or more of the following: identification information for the patient; socioeconomic information for the patient; medical history for the patient; treatment history for the patient; medical directives for the patient; one or more physiological measurements taken from the patient; virtual care solution usage information; a technology affinity measured for the patient; and feedback information from one or more historical or concurrent patients.

At a step 120, the method 100 includes extracting a plurality of dropout prediction features from the plurality of records obtained from the electronic patient records database. For example, in some embodiments, the plurality of dropout prediction features may include one or more of a response time from the care provider to a patient-initiated action, a history of challenges involving use of a virtual care solution by the patient, clinical trends for the patient, user interaction data for the patient, and/or RPM platform use data showing frequency of use of specific components over time, including but not limited to, login durations and login frequency.

At a step 130, the method 100 includes generating a dropout risk score for the patient based on the plurality of dropout prediction features. For example, the dropout risk score for the patient can be the likelihood that the patient will become non-adherent to a prescribed care plan within a particular timeframe. In embodiments, the dropout prediction engine comprises a trained dropout prediction model, which can be a machine learning model trained on a training dataset that comprises a plurality of medical records for a plurality of historical patients. In embodiments, the training dataset can include a plurality of medical records for a plurality of historical patients that have undergone remote patient monitoring. In further embodiments, the plurality of medical records can include information regarding any dropout conditions for the historical patients (e.g., if the historical patients ever dropped-out, the cause or causes contributing to the dropout, engagement actions taken, and the impact of such engagement actions, i.e., whether those engagement actions were successful). In particular embodiments, the dropout prediction model can be trained using generalized linear regression, extreme gradient boost, deep learning, and/or similar techniques.

In embodiments, generating a dropout risk score for the patient can include generating a risk score dropping out at several moments in the future (i.e., a certain future time measured from the time of the prediction). For example, when generating a dropout risk score for the patient, the dropout risk score may correspond to a particular time frame, such as a likelihood of dropout within the next 1 to 14 days. Put another way, at the step 130, the method 100 can include generating a first dropout risk score for the patient corresponding to a first future time, and generating at least a second dropout risk score for the patient corresponding to at least a second future time. In embodiments, the future time of interest may be, for example and without limitation, 1 day, 7 days, 14 days, 1 month, 2 months, and/or 3 months. In some embodiments, the first future time is between 1 and 14 days from a current time (i.e., a time of execution of step 130), while the second future time is between 1 and 6 months from the current time (i.e., the time of execution of step 130).

In a step 140, the method 100 includes predicting a potential dropout cause for the patient based on at least the dropout prediction features. In some embodiments, the potential dropout cause for the patient may be predicted by identifying the occurrence of a set of specific root causes known to influence dropout and/or by comparing the dropout prediction features with a repository of root causes stored in a data storage component of the dropout prediction system.

In some embodiments, the potential dropout cause may be determined by evaluating a feature value contribution for one or more dropout prediction features. For example, in particular embodiments, the feature value contributions for the plurality of prediction features can be determined using a Shapley values algorithm.

In some embodiments, the repository of root dropout causes can include, but is not limited to, long response times for the care provider to patient activity (e.g., response to an uploaded measurement) or requests for interaction (e.g., submission of a question via virtual care messaging platform), frequency and trend of occurrence of specific problems (e.g., login problems, problems uploading a measurement, problems using the chat features, problems accessing earlier measurements), usability problems manifesting as above-average times or effort needed to get certain tasks accomplished (e.g., time or number of clicks the patient uses to upload a measurement), clinical trends showing the patient is in a stable or unstable phase, subjective data and/or interaction data (e.g., assessing the experience of the patient with the virtual care system), virtual care system use data showing the frequency of use of specific components over time (e.g., the frequency of downloading healthcare content or articles, the frequency and type of uploaded measurements), and/or the like.

In embodiments, predicting a potential dropout cause for the patient can include predicting a potential dropout cause for the patient at several moments in the future (i.e., a certain future time measured from the time of the prediction). For example, when predicting a potential dropout cause for the patient, the potential dropout cause may correspond to a particular time frame, such as a potential dropout cause within the next 1 to 14 days. Put another way, at the step 140, the method 100 can include predicting a potential dropout cause for the patient corresponding to a first future time, and predicting at least a second potential dropout cause for the patient corresponding to at least a second future time. In embodiments, the future time of interest may be, for example and without limitation, 1 day, 7 days, 14 days, 1 month, 2 months, and/or 3 months. In some embodiments, the first future time is between 1 and 14 days from a current time (i.e., a time of execution of step 130), while the second future time is between 1 and 6 months from the current time (i.e., the time of execution of step 130).

In a step 150, the method 100 includes determining a recommended engagement action tailored to the patient based on the generated dropout risk score and the predicted root dropout cause. In embodiments, the one or more recommended engagement actions are intended to prevent dropout of the patient from the RPM program provided by the care provider.

In embodiments, determining one or more recommended engagement actions can include selecting an engagement action from a library of interventions or engagement actions stored in a data storage component of the dropout prediction system. For example, the data storage component of the dropout prediction system can store a library of interventions (also referred to as engagement actions) that are possible given the deployment environment of the RPM platform, and/or the characteristics of the relevant patient or patients. In embodiments, the library of possible engagement actions can include options for the care provider to contact the relevant patient or patients (e.g., via a messaging system of the RPM platform, by telephone, email, mail, etc.), distribution of a survey to the patient or patients, a request for an in-person appointment, and/or the like.

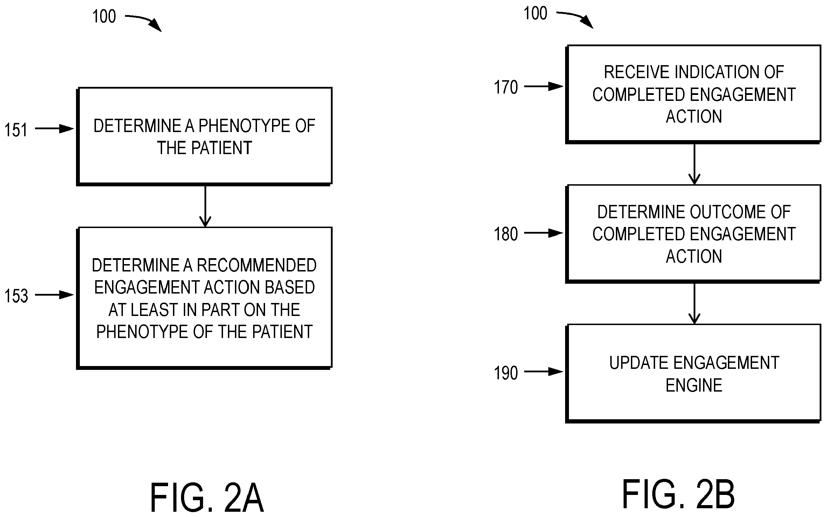

In some embodiments, determining one or more recommendation engagement actions can include one or more intermediate steps. For example, with reference to FIG. 2A, determining one or more recommended engagement actions can include, in a first step 151, determining a phenotype for the relevant patient or patients, and then in a second step 153, determining an engagement action based on the patient phenotype. In particular, a patient phenotype can be a classification or subgroup that the patient may be sorted into based on one or more similar characteristics. For example, if the records for a particular patient indicates a good comfortability with technology, then the patient may be classified into a subgroup of patients with similar comfortability. In turn, the engagement action that is then recommended may be based on that phenotype (i.e., comfortability with technology), for example, by recommending a technology-based engagement action. In contrast, for patients with a phenotype indicating less comfortability with technology, a care provider might receive a different recommended engagement action to be implemented through telephone, in-person appointments, and/or the like.

It should be appreciated, however, that the patient phenotype may be determined or generated based on other factors, including but not limited to, health factors for the patient, one or more of the dropout prediction features, socioeconomic conditions of the patient, and/or the like, including combinations of such factors. In particular embodiments, similar patients and/or similar patient cohorts may be identified through clustering of historical patients with records stored in or accessible to the database. In some embodiments, the identification of similar patients and/or similar patient cohorts may be performed using an unsupervised hierarchical clustering algorithm, where subsequent and/or new patients are assigned to a cluster based on some distance measure along the feature space. In embodiments, the feature space may comprise one or more of the dropout prediction features.

In embodiments, determining one or more recommended engagement actions can also include determining one or more recommended engagement actions to take or perform over a period of time in the future (i.e., a certain future time measured from the time of the prediction). For example, the one or more recommended engagement actions may include an immediate intervention, a short-term intervention, and/or a long-term intervention. In further embodiments, determining one or more recommended interventions can include determining one or more recommended interventions for each predicted root dropout cause and/or likelihood of dropout for the patient.

In a step 160, the method 100 includes presenting the one or more recommended engagement actions determined in step 150 to the care provider. For example, in some embodiments, the one or more recommended engagement actions may be presented to a member of a care team associated with the patient for whom the dropout risk score and subsequent recommended engagement actions were generated. In embodiments, this may be a nurse, a nurse practitioner, a physician, a doctor, a clinician, a dietitian, a wellness coach, and/or the like. In embodiments, the one or more recommended engagement actions may be presented via a care provider interface of the dropout prediction system 304.

In embodiments, the method 100 can further include one or more steps to gauge the impact of an engagement action and update its recommendations accordingly. More specifically, with reference to FIG. 2B, the method 100 can further include: in a step 170, receiving an indication that one or more recommended engagement actions were completed; in a step 180, determining an outcome of one of more of the recommended engagement actions; and, in a step 190, updating engagement engine based on the impact or outcome determined for the one or more recommended engagement actions.

In embodiments, step 170 of the method 100 may comprise creating an electronic record associated with the patient in the dropout prediction database of the dropout prediction system, wherein the record comprises any information about any engagement actions taken to prevent dropout of the patient from occurring. In embodiments, the information stored can comprise what engagement actions were taken, by whom, and how such actions were completed (i.e., in what form), as well as when the patient received or acknowledged the engagement action and the reaction of the patient. As such, in specific embodiments, step 170 includes receiving or collecting such information and storing it in the dropout prediction database.

In embodiments, step 180 of the method 100 includes determining an outcome of the one or more engagement actions that were taken in relation to a particular patient. That is, step 180 may comprise determining whether the engagement actions taken by the care provider were successful in preventing dropout of the patient from the RPM program. In some embodiments, the impact or outcome may be determined in relation to one or more predicted dropout causes. For example, if a patient was at risk of dropping out of an RPM program due to a difficulty in using the virtual care platform and a recommended engagement action involving a coached training session and/or online training was completed, the outcome may be determined as it relates to the difficulty in using the virtual care platform.

In embodiments, step 190 of the method 100 includes updating engagement engine based on the impact or outcome determined for the one or more recommended engagement actions. As a result, the engagement engine can stay up to date with the latest reported outcomes when recommending future engagement actions.

With reference to FIG. 3, a remote patient monitoring environment 300 is illustrated wherein a virtual care platform 302 having a dropout prediction system 304 is deployed in accordance with various aspects of the present disclosure. The dropout prediction system 302 may be embedded into the virtual care platform 302 or be separate from the virtual care platform 302 but in communication with virtual care platform 302 as it relates to a plurality of patients 306, 308, 310 enrolled in a remote patient monitoring through the virtual care platform 302. In some embodiments, the dropout prediction system 304 may be at least part of a large patient data management system (PDMS) and/or a virtual patient management solution or RPM system.

As described herein, the dropout prediction system 304 can be configured to perform one or more steps of the methods (e.g., method 100) described herein. In particular, the dropout prediction system 304 can be configured to predict a dropout risk for one or more patients 306, 308, 310 undergoing remote patient monitoring by a care provider 312, determine one or more potential dropout causes for one or more patients 306, 308, 310, and determine one or more recommended engagement actions for one or more patients 306, 308, 310.

In the example of FIG. 3, the patients 306, 308, 310 are undergoing a remote patient monitoring protocol being administered by a care provider 312. These patients 306, 308, 310 may be under the same protocol or different protocols, for example, for differing conditions (e.g., respiratory issues in the case of a first patient 306, cardiac issues in the case of a second patient 308, and/or weight issues in the case of a third patient 310). As such, the dropout prediction system 304 may be used to monitor such patients 306, 308, 310 for potential dropout and dropout causes.

In embodiments, the dropout prediction system 304 may comprise an electronic patient records database 314 that stores a plurality of medical records for a plurality of patients, including but not limited to the patients 306, 308, 310. That is, the electronic patient records database 314 can store medical records for a plurality of patients can include patients currently active in an RPM program administered by a care provider 408, but can also include historical patients (i.e., patients no longer active in an RPM program).

In embodiments, the plurality of records may include identification information for one or more patients, medical histories for one or more patients, treatment histories for one or more patients, medical directives for one or more patients, and/or physiological measurements or lab results for one or more patients. In some embodiments, the plurality of records may include self-reported data on medical conditions (existing or new), physical health, and mental health for one or more patients. Put another way, the plurality of records can include medical data generated and reported by one or more patients, including but not limited to, updated physiological measurements and test results.

In further embodiments, the plurality of records can also include patterns of use of the virtual care system used in the patient's RPM program, including but not limited to, virtual care platforms, virtual health assistants, electronic and/or outpatient medical devices, virtual visit systems, medical appointment scheduling systems, and the like. In embodiments, the plurality of records include patterns of medical data (e.g., test results, hospital visits, provider interactions), as well as other engagement-related features such as medication adherence. The plurality of records may also include subjective data that is collected, self-reported, and/or assessed over time, including but not limited to, the patient's comfortability and/or affinity with technology, feedback from one or more historical or concurrent patients, and the like.

The dropout prediction system 304 can also comprise a dropout prediction database 316 comprising a repository of root dropout causes as well as a library of engagement actions. In some embodiments, the repository of root dropout causes can include, but are not limited to, long response times for the care provider to patient activity (e.g., response to an uploaded measurement) or requests for interaction (e.g., submission of a question via virtual care messaging platform), frequency and trend of occurrence of specific problems (e.g., login problems, problems uploading a measurement, problems using the chat features, problems accessing earlier measurements), usability problems manifesting as above-average times or effort needed to get certain tasks accomplished (e.g., time or number of clicks the patient uses to upload a measurement), clinical trends showing the patient is in a stable or unstable phase, subjective data and/or interaction data (e.g., assessing the experience of the patient with the virtual care system), and/or virtual care system use data showing the frequency of use of specific components over time (e.g., the frequency of downloading healthcare content or articles, the frequency and type of uploaded measurements).

In still further embodiments, the library of interventions (also referred to as engagement actions) can record possible engagement actions given the deployment environment 300 of the RPM platform, and/or the characteristics of the relevant patient or patients 306, 308, 310. In embodiments, the library of possible engagement actions can include options for a care team member of the care provider to contact the relevant patient or patients (e.g., via a messaging system of the RPM platform, by telephone, email, mail, etc.), distribution of a survey to the patient or patients, a request for an in-person appointment, and/or the like.

As described in more detail below, each of the patients under remote monitoring (e.g., patients 306, 308, 310) may interact with the remote patient monitoring platform 302 via one or more different patient devices 318, 320, 322, including but not limited to, a patient monitor, a desktop computer, a laptop computer, a mobile phone, a tablet, and/or the like. The dropout prediction system 304 may provide, via the remote patient monitoring platform 302 in communication with such devices 318, 320, 322, a patient interface whereby the care provider 312 may complete one or more engagement actions. In embodiments, the care provider 312 may also be in communication with the remote patient monitoring platform 302 and the dropout prediction system 304 via a care provider device 324.

More particularly, with reference to FIG. 4, various components of the dropout prediction system 304 are illustrated in accordance with aspects of the present disclosure. As mentioned above, the dropout prediction system 304 can comprise: an electronic patient records database 314 comprising a plurality of medical records for one or more patients under remote monitoring by the care provider; a dropout prediction database 316 comprising a repository of root dropout causes and a library of engagement actions; a dropout prediction engine 414 configured to generate one or more dropout risk scores for one or more patients; an engagement recommendation engine 416 configured to determine one or more potential dropout cause and one or more recommended engagement actions for one or more patients; a care provider interface 422 configured to present one or more dropout risk scores generated for one or more patients, the one or more potential dropout causes for one or more patients, and/or the one or more recommended engagement actions for one or more patients; and one or more processors 402 configured to perform one or more steps of the method (e.g., method 100) described herein.

In particular embodiments, the dropout prediction system 304 may comprise one or more processors 402, machine-readable memory 404, a user interface 406, and/or a communications interface 408, all of which may be interconnected and/or communication through a system bus 410 containing conductive circuit pathways through which instructions (e.g., machine-readable signals) may travel to effectuate communication, tasks, storage, and the like.

The one or more processors 402 may be configured to perform one or more steps of the methods described herein, including but not limited to, the following: obtain, from the electronic patient records database 314, a plurality of medical records for at least a first patient; extract, from the plurality of medical records, a plurality of dropout prediction features for at least the first patient; generate, using a dropout prediction engine 414, a dropout risk score for at least the first patient based on the plurality of dropout prediction features; determine, using an engagement engine 416, a potential dropout cause for at least the first patient based on at least the plurality of dropout prediction features; determine, using the engagement recommendation engine 414, a recommended engagement action, wherein the recommended engagement action is intended to prevent dropout of at least the first patient from remote monitoring by the care provider; and present, via a care provider interface 422, present the dropout risk score generated for at least the first patient, the potential dropout cause for at least the first patient, and/or the recommended engagement action for at least the first patient.

In some examples, the one or more processors 402 may include a high-speed data processor adequate to execute the program components described herein and/or various specialized processing units as may be known in the art. In some examples, the one or more processors 402 may be a single processor, multiple processors, or multiple processor cores on a single die.

In some examples, the communications interface 408 can include a network interface configured to connect the dropout prediction system 304 to a communications network 424, an input/output ("I/O") interface configured to connect and communicate with one or more peripheral devices, a memory interface configured to accept, communication, and/or connect to a number of machine-readable memory devices, and the like. For example, the communications interface 408 may operatively connect the dropout prediction system 304 to a communications network 424, which can include a direct interconnection, the Internet, a local area network ("LAN"), a metropolitan area network ("MAN"), a wide area network ("WAN"), a wired or Ethernet connection, a wireless connection, and similar types of communications networks, including combinations thereof. In some examples, dropout prediction system 304 may communicate with one or more remote / cloud-based servers (e.g., the electronic medical records database 314), cloud-based services, and/or remote devices via the communications network 424.

The memory 404 can be variously embodied in one or more forms of machine-accessible and machine-readable memory. In some examples, the memory 404 includes a storage device that comprises one or more types of memory. For example, a storage device can include, but is not limited to, a non-transitory storage medium, a magnetic disk storage, an optical disk storage, an array of storage devices, a solid-state memory device, and the like, including combinations thereof.

Generally, the memory 404 is configured to store data / information and instructions that, when executed by the one or more processors 404, causes the dropout prediction system 304 to perform one or more tasks. In particular examples, the memory 404 includes a dropout prediction package 426 that causes the dropout prediction system 304 to perform one or more steps of the methods described herein.

In embodiments, the dropout prediction package 426 comprises a collection of program components, database components, and/or data. Depending on the particular implementation, the dropout prediction package 426 may include software components, hardware components, and/or some combination of both hardware and software components. The dropout prediction package 426 may include one or more software packages configured to predict a likelihood of dropout for a patient and/or root dropout causes. These software packages may be incorporated into, loaded from, loaded onto, or otherwise operatively available to and from the dropout prediction system 400. In some examples, the dropout prediction package 426 and/or one or more individual software packages may be stored in a local storage device 404. In other examples, the dropout prediction package 426 and/or one or more individual software packages may be loaded onto and/or updated from a remote server via the communications interface 424.

In particular embodiments, the dropout prediction package 426 can include, but is not limited to, instructions having a data collection component 412, a dropout prediction engine 414, an engagement engine 416, a care provider user interface (UI) component 418, and/or a patient user interface (UI) component 420. These components may be incorporated into, loaded from, loaded onto, or otherwise operatively available to and from the dropout prediction system 304.

In embodiments, the data collection component 412 can be a stored program component that is executed by at least one processor, such as the one or more processors 402 of the dropout prediction system 304. In particular, the data collection component 412 can be configured to interface with an electronic medical records database 314 in order to obtain a plurality of records for one or more patients, as described herein. That is, the data collection component 412 may be configured to request, receive, and/or otherwise obtain a plurality of medical records for one or more patients. In some embodiments, one or more of the patients may be historical patients. In other embodiments, one or more of the patients may be current RPM program patients 306, 308, 310. In still further embodiments, the data collection component 412 may obtain a plurality of records for a combination of historical and/or current RPM program patients 306, 308, 310.

In further embodiments, the data collection component 412 can be configured to extract a plurality of different predefined dropout prediction features for a patient from the plurality of obtained records, as described herein. For example, the data collection component 412 can be configured to extract predefined dropout prediction features from the plurality of records obtained from an electronic medical records database 314 using natural language processing and/or a machine learning algorithm.

In embodiments, the dropout prediction engine 414 can be a stored program component that is executed by at least one processor, such as the one or more processors 404 of the dropout prediction system 304. In embodiments, the dropout prediction engine 414 is configured to generate a dropout risk score for the patient based on these plurality of dropout prediction features. For example, the dropout risk score for the patient can be the likelihood that the patient will become non-adherent to a prescribed care plan within a particular timeframe. In embodiments, the dropout prediction engine comprises a trained dropout prediction model, which can be a machine learning model trained on a training dataset that comprises a plurality of medical records for a plurality of historical patients. In particular embodiments, the dropout prediction model can be trained using generalized linear regression, extreme gradient boost, deep learning, and/or similar techniques.

In embodiments, the engagement engine 416 can be a stored program component that is executed by at least one processor, such as the one or more processors 404 of the dropout prediction system 304. In particular, the engagement engine 414 can be configured to predict a potential dropout cause for one or more patients based on at least the dropout prediction features. In some embodiments, the potential dropout cause for the patient may be predicted by identifying the occurrence of a set of specific root causes known to influence dropout and/or by comparing the dropout prediction features with a repository of root causes stored in a data storage component (e.g., the dropout prediction database 316 of the memory 404 of the dropout prediction system 304). In some embodiments, the repository of root dropout causes can include, but are not limited to, long response times for the care provider to patient activity (e.g., response to an uploaded measurement) or requests for interaction (e.g., submission of a question via virtual care messaging platform), frequency and trend of occurrence of specific problems (e.g., login problems, problems uploading a measurement, problems using the chat features, problems accessing earlier measurements), usability problems manifesting as above-average times or effort needed to get certain tasks accomplished (e.g., time or number of clicks the patient uses to upload a measurement), clinical trends showing the patient is in a stable or unstable phase, subjective data and/or interaction data (e.g., assessing the experience of the patient with the virtual care system), and/or virtual care system use data showing the frequency of use of specific components over time (e.g., the frequency of downloading healthcare content or articles, the frequency and type of uploaded measurements).

As described above, the potential dropout cause may be determined by evaluating a feature value contribution for one or more dropout prediction features. For example, in particular embodiments, the feature value contributions for the plurality of prediction features can be determined using a Shapley values algorithm. In further embodiments, predicting a potential dropout cause for the patient can include predicting a potential dropout cause for the patient at several moments in the future (i.e., a certain future time measured from the time of the prediction). For example, when predicting a potential dropout cause for the patient, the potential dropout cause may correspond to a particular time frame, such as a potential dropout cause within the next 1 to 14 days. Put another way, at the step 140, the method 100 can include predicting a potential dropout cause for the patient corresponding to a first future time, and predicting at least a second potential dropout cause for the patient corresponding to at least a second future time. In embodiments, the future time of interest may be, for example and without limitation, 1 day, 7 days, 14 days, 1 month, 2 months, and/or 3 months. In some embodiments, the first future time is between 1 and 14 days from a current time (i.e., a time of execution of step 130), while the second future time is between 1 and 6 months from the current time (i.e., the time of execution of step 130).

In further embodiments, the engagement engine 416 may be configured to determine a phenotype for the relevant patient or patients, and then determine an engagement action based on the patient phenotype. In particular, a patient phenotype can be a classification or subgroup that the patient may be sorted into based on one or more similar characteristics. For example, if the records for a particular patient indicates a good comfortability with technology, then the patient may be classified into a subgroup of patients with similar comfortability. In turn, the engagement action that is then recommended may be based on that phenotype (i.e., comfortability with technology), for example, by recommending a technology-based engagement action. In contrast, for patients with a phenotype indicating less comfortability with technology, a care provider might receive a recommended engagement action implemented through telephone, in-person appointments, and/or the like.

It should be appreciated, however, that the patient phenotype may be determined or generated based on other factors, including but not limited to, health factors for the patient, one or more of the dropout prediction features, socioeconomic conditions of the patient, and/or the like, including combinations of such factors. In particular embodiments, similar patients and/or similar patient cohorts may be identified through clustering of historical patients with records stored in or accessible to the database. In some embodiments, the identification of similar patients and/or similar patient cohorts may be performed using an unsupervised hierarchical clustering algorithm, where subsequent and/or new patients are assigned to a cluster based on some distance measure along the feature space. In embodiments, the feature space may comprise one or more of the dropout prediction features.

In some embodiments, the engagement component 416 can also be configured to create records in the dropout prediction database 316 that stores information about any engagement actions taken to prevent a patient dropout from occurring. In further embodiments, the intervention component 416 may be configured to determine an impact or outcome of the one or more engagement actions that are taken. For example, in some embodiments, an impact of taking one or more recommended engagement actions may be determined for each of the one or more dropout prediction features used to determine the likelihood of dropout for the patient. Then, the engagement engine 416 may be updated to provide better recommendations in the future.

In embodiments, the care provider UI component 418 can be a stored program component that is executed by at least one processor, such as the one or more processors 402 of the dropout prediction system 304. In particular, the care provider UI component 418 can be configured operate a care provider user interface 422 in order to present information to a care provider 312, as described herein. In some embodiments, the care provider UI component 418 can include a programmable processor, such as a graphics processing units (GPU), which is specialized for rendering images on a monitor or display screen of a user device 324. In other words, the user device 324 may be configured, via a care provider UI component 418, to provide or otherwise present a likelihood of dropout and/or root dropout cause generated for one or more patients.

In embodiments, the patient UI component 420 can be a stored program component that is executed by at least one processor, such as the one or more processors 402 of the dropout prediction system 304. In particular, the patient UI component 420 can be configured operate a patient device 318, 320, 322 in order to present information to a corresponding patient 306, 308, 310, as described herein. In some embodiments, the patient UI component 420 can include a programmable processor, such as a graphics processing units (GPU), which is specialized for rendering images on a monitor or display screen of a user device 318, 320, 322. In particular embodiments, the user devices 318, 320, 322 may be configured, via a patient UI component 420, to provide or otherwise present one or more outreach / intervention attempts to the patient corresponding patients 306, 308, 310.

The dropout prediction system 304 may also include an operating system component 428, which may be stored in the memory 404. The operating system component 428 may be an executable program facilitating the operation of the dropout prediction system 304. Typically, the operating system component 428 can facilitate access of the communications interface 408, and can communicate with other components of the dropout prediction system 304, including but not limited to, the user interface 422, the memory 404, and/or the electronic medical records database 314.

It should be appreciated that all combinations of the foregoing concepts (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein. It should also be appreciated that terminology explicitly employed herein that also may appear in any disclosure incorporated by reference should be accorded a meaning most consistent with the particular concepts disclosed herein.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of."

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

As used herein, although the terms first, second, third, etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

Unless otherwise noted, when an element or component is said to be "connected to," "coupled to," or "adjacent to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of' and "consisting essentially of' shall be closed or semi-closed transitional phrases, respectively.

It should also be understood that, unless clearly indicated to the contrary, in any methods claimed herein that include more than one step or act, the order of the steps or acts of the method is not necessarily limited to the order in which the steps or acts of the method are recited.

The above-described examples of the described subject matter can be implemented in any of numerous ways. For example, some aspects can be implemented using hardware, software or a combination thereof. When any aspect is implemented at least in part in software, the software code can be executed on any suitable processor or collection of processors, whether provided in a single device or computer or distributed among multiple devices/computers.

The present disclosure can be implemented as a system, a method, and/or a computer program product at any possible technical detail level of integration. The computer program product can include a computer readable storage medium (or media) having computer readable program instructions thereon for causing a processor to carry out aspects of the present disclosure.

The computer readable storage medium can be a tangible device that can retain and store instructions for use by an instruction execution device. The computer readable storage medium can be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium comprises the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network and/or a wireless network. The network can comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device.

Computer readable program instructions for carrying out operations of the present disclosure can be assembler instructions, instruction-set-architecture (ISA) instructions, machine instructions, machine dependent instructions, microcode, firmware instructions, state-setting data, configuration data for integrated circuitry, or either source code or object code written in any combination of one or more programming languages, comprising an object oriented programming language such as Smalltalk, C++, or the like, and procedural programming languages, such as the "C" programming language or similar programming languages. The computer readable program instructions can execute entirely on the user's computer, partly on the user's computer, as a stand-alone software package, partly on the user's computer and partly on a remote computer or entirely on the remote computer or server. In the latter scenario, the remote computer can be connected to the user's computer through any type of network, comprising a local area network (LAN) or a wide area network (WAN), or the connection can be made to an external computer (for example, through the Internet using an Internet Service Provider). In some examples, electronic circuitry comprising, for example, programmable logic circuitry, field-programmable gate arrays (FPGA), or programmable logic arrays (PLA) can execute the computer readable program instructions by utilizing state information of the computer readable program instructions to personalize the electronic circuitry, in order to perform aspects of the present disclosure.

Aspects of the present disclosure are described herein with reference to flowchart illustrations and/or block diagrams of methods, apparatus (systems), and computer program products according to examples of the disclosure. It will be understood that each block of the flowchart illustrations and/or block diagrams, and combinations of blocks in the flowchart illustrations and/or block diagrams, can be implemented by computer readable program instructions.

The computer readable program instructions can be provided to a processor of a, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions can also be stored in a computer readable storage medium that can direct a computer, a programmable data processing apparatus, and/or other devices to function in a particular manner, such that the computer readable storage medium having instructions stored therein comprises an article of manufacture comprising instructions which implement aspects of the function/act specified in the flowchart and/or block diagram or blocks.

The computer readable program instructions can also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The flowchart and block diagrams in the Figs illustrate the architecture, functionality, and operation of possible implementations of systems, methods, and computer program products according to various examples of the present disclosure. In this regard, each block in the flowchart or block diagrams can represent a module, segment, or portion of instructions, which comprises one or more executable instructions for implementing the specified logical function(s). In some alternative implementations, the functions noted in the blocks can occur out of the order noted in the Figs. For example, two blocks shown in succession can, in fact, be executed substantially concurrently, or the blocks can sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

Other implementations are within the scope of the following claims and other claims to which the applicant can be entitled.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

## Claims

1. A method (100) for predicting dropout risk for a patient under remote monitoring using a dropout prediction system (304), the method comprising:
obtaining (110), from an electronic patient records database (314), a plurality of medical records for a patient under remote monitoring by a care provider;
extracting (120), from the plurality of medical records for the patient under remote monitoring by the care provider, a plurality of dropout prediction features for the patient;
generating (130), using a dropout prediction engine (414), a dropout risk score for the patient based on the plurality of dropout prediction features;
determining (140), using an engagement recommendation engine (416), a potential dropout cause based on at least the plurality of dropout prediction features;
determining (150), using the engagement recommendation engine (416), a recommended engagement action, wherein the recommended engagement action is intended to prevent dropout of the patient from remote monitoring by the care provider; and
presenting (160), via a care provider interface (422), the recommended engagement action to a care team member of the care provider.

2. The method (100) of claim 1, wherein the dropout prediction engine (414) comprises a trained dropout prediction model, the trained dropout prediction model being trained by a machine learning algorithm on a training dataset that comprises a plurality of medical records for a plurality of historical patients.

3. The method (100) of claim 1, wherein the potential dropout cause is determined by evaluating a feature value contribution for one or more dropout prediction features, the feature value contributions being determined using a Shapley values algorithm.

4. The method (100) of claim 1, wherein the plurality of medical records for the patient under remote monitoring include at least one of: identification information for the patient; socioeconomic information for the patient; medical history for the patient; treatment history for the patient; medical directives for the patient; and one or more physiological measurements taken from the patient.

5. The method (100) of claim 4, wherein the plurality of medical records for the patient under remote monitoring further includes at least one of: virtual care solution usage information; a technology affinity measured for the patient; and feedback information from one or more historical or concurrent patients.

6. The method (100) of claim 1, wherein the dropout risk score generated for the patient is a likelihood that the patient will fail to meet a participation threshold set by a third party.

7. The method (100) of claim 6, wherein the third party is an insurance company and the participation threshold determines whether the remote monitoring of the patient is covered under an insurance plan associated with the patient.

8. The method (100) of claim 1, further comprising:
determining (151) a phenotype for the patient under remote monitoring based on one or more of the dropout prediction features, the dropout risk score generated for the patient, and the potential dropout cause determined for the patient, wherein the phenotype for the patient indicates a subgroup of similar historical patients;
wherein the recommended engagement action is determined (153) at least in part based on the phenotype for the patient.

9. A dropout prediction system (304) configured to predict a dropout risk for one or more patients undergoing remote patient monitoring by a care provider, the system (304) comprising:
an electronic patient records database (314) comprising a plurality of medical records for the one or more patients under remote monitoring by the care provider;
a dropout prediction database (316) comprising a repository of root dropout causes and a library of engagement actions;
a dropout prediction engine (414) configured to generate one or more dropout risk scores for the one or more patients;
an engagement recommendation (416) engine configured to determine one or more potential dropout cause and one or more recommended engagement actions for the one or more patients;
a care provider interface (422) configured to present one or more dropout risk scores generated for the one or more patients, the one or more potential dropout causes for the one or more patients, and/or the one or more recommended engagement actions for the one or more patients; and
one or more processors (402) configured to:
obtain, from the electronic patient records database (314), a plurality of medical records for at least a first patient;
extract, from the plurality of medical records, a plurality of dropout prediction features for at least the first patient;
generate, using the dropout prediction engine (414), a dropout risk score for at least the first patient based on the plurality of dropout prediction features;
determine, using the engagement recommendation engine (416), a potential dropout cause for at least the first patient based on at least the plurality of dropout prediction features;
determine, using the engagement recommendation engine (416), a recommended engagement action, wherein the recommended engagement action is intended to prevent dropout of at least the first patient from remote monitoring by the care provider; and
present, via the care provider interface (422), present the dropout risk score generated for at least the first patient, the potential dropout cause for at least the first patient, and/or the recommended engagement action for at least the first patient.

10. The dropout prediction system (304) of claim 9, wherein the dropout prediction engine (414) comprises a trained dropout prediction model, the trained dropout prediction model being trained by a machine learning algorithm on a training dataset that comprises a plurality of medical records for a plurality of historical patients.

11. The dropout prediction system (304) of claim 9, wherein each potential dropout cause is determined by evaluating a feature value contribution for one or more dropout prediction features, the feature value contributions being determined using a Shapley values algorithm.

12. The dropout prediction system (304) of claim 9, wherein the plurality of records for the patient under remote monitoring by the care provider include at least one of: identification information for the patient; socioeconomic information for the patient; medical history for the patient; treatment history for the patient; medical directives for the patient; and one or more physiological measurements taken from the patient.

13. The dropout prediction system (304) of claim 9, wherein the plurality of records for the patient under remote monitoring by the care provider further includes at least one of: virtual care solution usage information; a technology affinity measured for the patient; and feedback information from one or more historical or concurrent patients.

14. The dropout prediction system (304) of claim 9, wherein the engagement recommendation engine (416) is further configured to determine a phenotype for the one or more patients, each phenotype indicating a subgroup of similar historical patients; and
wherein the one or more processors (402) are further configured to:
determine, using the engagement recommendation engine (416), a phenotype for at least the first patient based on one or more of the dropout prediction features of at least the first patient, the dropout risk score generated for at least the first patient, and the potential dropout cause determined for at least the first patient; and
determine a recommended engagement action based at least in part on the phenotype determined for at least the first patient.

15. The dropout prediction system (304) of claim 9, wherein the dropout risk score generated for at least the first patient is a likelihood that the patient will fail to meet a participation threshold set by a third party, the third party being an insurance company and the participation threshold determining whether the remote monitoring of at least the first patient is covered under an insurance plan associated with at least the first patient.
